(19) **Europäisches Patentamt / European Patent Office / Office européen des brevets**

(11) **EP 4 613 257 A1**

(12) **EUROPEAN PATENT APPLICATION**
published in accordance with Art. 153(4) EPC

(43) Date of publication:
**10.09.2025 Bulletin 2025/37**

(21) Application number: **23885674.4**

(22) Date of filing: **27.10.2023**

(51) International Patent Classification (IPC):
*A61K 8/34* (2006.01)     *A61Q 1/14* (2006.01)
*C11D 1/66* (2006.01)     *C11D 1/68* (2006.01)
*C11D 3/20* (2006.01)

(52) Cooperative Patent Classification (CPC):
A61K 8/34; A61Q 1/14; C11D 1/66; C11D 3/20;
C11D 3/2003

(86) International application number:
**PCT/JP2023/038902**

(87) International publication number:
**WO 2024/095918 (10.05.2024 Gazette 2024/19)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **31.10.2022 JP 2022175105**

(71) Applicant: **Kao Corporation**
**Chuo-ku,**
**Tokyo 103-8210 (JP)**

(72) Inventors:
- **TSUDA, Hiroko**
  **Tokyo 131-8501 (JP)**
- **UEYAMA, Chihiro**
  **Tokyo 131-8501 (JP)**
- **KAGAYA, Mariko**
  **Tokyo 131-8501 (JP)**
- **NAGASAKI, Yuko**
  **Tokyo 131-8501 (JP)**

(74) Representative: **Hoffmann Eitle**
**Patent- und Rechtsanwälte PartmbB**
**Arabellastraße 30**
**81925 München (DE)**

(54) **CLEANING AGENT COMPOSITION**

(57)     A cleansing composition containing the following components (A), (B), and (C): (A) 7 to 35 mass% of a nonionic surfactant containing (a1) a multi-chain type nonionic surfactant which has two or more of at least one selected from the group consisting of an unsaturated chain hydrocarbon group and a saturated branched chain hydrocarbon group, and having an HLB value (a mixed HLB value when two or more nonionic surfactants are contained) of from 9.5 to 12.5; (B) 60 to 90 mass% of an oil phase having a viscosity at 25°C of 11 mPa·s or less; and (C) 0.05 to 8 mass% of one or more selected from the group consisting of water, an alcohol having 2 or 3 carbon atoms, and a dihydric alcohol.

**EP 4 613 257 A1**

**Description**

Field of the Invention

The present invention relates to a cleansing composition.

Background of the Invention

Oil-based makeup removers are widely accepted in the market as makeup removers with strong cleansing power. Oil-based makeup removers are formulations prepared by combining an oil agent which exhibits cleansing power, and a surfactant which emulsifies the oil agent for rinsing. Many studies have been conducted to improve use impression unique to oil, decrease in cleansing power due to water contamination, and other issues.

For example, Patent Literature 1 describes that an oily cleansing cosmetic containing a di-branched fatty acid polyethylene glycol, an oil component, and water is excellent in fresh feeling, and is further excellent in cleansing effect, ease of removal, ease of application, and storage stability.

Further, Patent Literature 2 describes that a transparent liquid oily composition containing three types of nonionic surfactants with a specific range of IOBs can be used as a cleansing cosmetic in environments where water is present, such as bathrooms and washbasins.

(Patent Literature 1) JP-A-2002-241224
(Patent Literature 2) JP-A-2004-238376

Summary of the Invention

The present invention relates to a cleansing composition comprising the following components (A), (B), and (C):

(A) 7 to 35 mass% of a nonionic surfactant comprising (a1) a multi-chain type nonionic surfactant which has two or more of at least one selected from the group consisting of an unsaturated chain hydrocarbon group and a saturated branched chain hydrocarbon group, and having an HLB value (a mixed HLB value when two or more nonionic surfactants are contained) of from 9.5 to 12.5;
(B) 60 to 85 mass% of an oil phase having a viscosity at 25°C of 11 mPa·s or less; and
(C) 0.05 to 8 mass% of one or more selected from the group consisting of water, an alcohol having 2 or 3 carbon atoms, and a dihydric alcohol.

Brief Description of Drawing

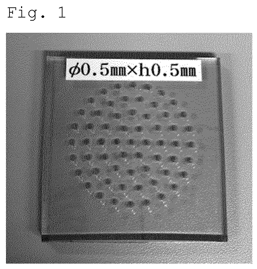

Fig. 1 illustrates a vinyl chloride sheet used to evaluate the removal of makeup falling into deep holes in Examples.

Detailed Description of the Invention

In recent years, due to global warming and the need to wear masks during infectious disease outbreaks, long-lasting makeup which does not come off easily has become popular. Long-lasting makeup cosmetics commonly use, for example, a technique of incorporating an oil-soluble silicone resin, such as trimethylsiloxysilicate (TMS), to form a hard, water-repellent cosmetic coating film on the skin surface, not only providing resistance to sweat and sebum but also physically preventing makeup from coming off. Relating to makeup removers, there is an increasing awareness of the need to avoid rubbing the skin, and user behavior has changed toward low friction for a short period of time. Under such circumstances, even with oil-based makeup removers, which are considered to have high cleansing power, makeup in the recessed areas of facial features such as around the eyes and nose, or in the pores, may not be completely removed and remain. This has been one of the user's complaints.

Thus, with conventional compositions, it was difficult to completely remove hard-to-remove stains, such as long-lasting foundations and other cosmetics containing film-forming agents, from facial surfaces with irregularities of various sizes by a small physical force.

The problem of not being able to completely remove makeup from recessed areas which are inaccessible to the human hand is because conventional makeup removers are based on the premise that makeup and an agent are mixed thoroughly by physical force and then rinsed off. Therefore, the current behavior of not rubbing the skin creates a disadvantageous situation in terms of mixing the agent and makeup, and causes a dysfunction in which the agent is rinsed off while makeup is not loosened sufficiently. That is, the more people try to remove makeup without rubbing the skin, the more anxiety and stress they feel about remaining makeup.

The present invention provides a composition wherein a nonionic surfactant containing a specific multi-chain type nonionic surfactant and having a specific HLB, a low-viscosity oil agent, and a specific aqueous component are used in combination, thereby removing makeup from fine recesses such as pores, by a simple operation of applying it to the skin and rinsing it off against hard-to-remove stains, such as foundations containing film-forming agents.

The cleansing composition of the present invention can remove hard-to-remove stains, such as foundations containing film-forming agents, from small recesses such as pores, by spreading the agent over the stains and rinsing it off, without using physical force. With the present invention, users no longer need to blend the cleansing composition and makeup, and even the foundation in the pores can be properly removed without rubbing the skin.

The component (A) is a nonionic surfactant comprising (a1) a multi-chain type nonionic surfactant which has two or more of at least one selected from the group consisting of an unsaturated chain hydrocarbon group and a saturated branched chain hydrocarbon group, and having an HLB value (a mixed HLB value when two or more nonionic surfactants are contained) adjusted to from 9.5 to 12.5.

The HLB value as mentioned herein is an indicator showing hydrophile-lipophile balance, and is determined by the Griffin equation below in the present specification.

$$\text{HLB} = 20 \times \frac{\text{Molecular weight of hydrophilic group moiety}}{\text{Molecular weight of surfactant}}$$

When two or more nonionic surfactants are contained, the HLB value of the mixed surfactants is obtained by the arithmetic average of the HLB values of the respective nonionic surfactants based on their mass ratio.

$$\text{Mixed HLB} = \sum(\text{HLBx} \times \text{Wx})/\sum\text{Wx}$$

HLBx indicates the HLB value of a nonionic surfactant X.

Wx indicates the mass (g) of the nonionic surfactant X having a value of HLBx.

The multi-chain type nonionic surfactant which has two or more of at least one selected from the group consisting of an unsaturated chain hydrocarbon group and a saturated branched chain hydrocarbon group as the component (a1) is dissolved in an oil agent, penetrates into the makeup coating film entering the pores and other recesses together with the oil agent, and pulls the stains away from the skin or substrate during rinsing to disperse them in water.

The unsaturated chain hydrocarbon group is preferably a hydrocarbon group having 8 to 22 carbon atoms, more preferably a linear hydrocarbon group having 12 to 18 carbon atoms, and more preferably an oleyl group. The saturated branched chain hydrocarbon group is preferably a hydrocarbon group having 8 to 22 carbon atoms, more preferably a hydrocarbon group having 12 to 18 carbon atoms, and more preferably an isostearoyl group.

It is important for the component (a1) to have lipophilic properties from the viewpoint of dissolving in the oil agent and spontaneously penetrating into makeup and other oily stains. On the other hand, it is also necessary for the component (a1) to have moderate hydrophilic properties from the viewpoint of spontaneous dispersion and rinsing by simply applying water without rubbing. In order to achieve the former properties, it is advantageous to have a structure with multiple long-chain alkyls. From the latter point of view, it is preferable to select a component with a relatively high HLB value so that it will exhibit affinity when in contact with water.

For this reason, the multi-chain type nonionic surfactant as the component (a1) preferably has an HLB value of from 7 to 13, and more preferably of from 8 to 12.

The component (a1) is also preferably selected from the group consisting of a POE sorbitan fatty acid ester, a POE sorbitol fatty acid ester, a POE glyceryl ether fatty acid ester, and a POE fatty acid ester, each of which has 2 to 4 oleyl groups or isostearoyl groups, in terms of high versatility and ease of availability.

The nonionic surfactant as the component (A) can be combined with a nonionic surfactant other than the component (a1).

The nonionic surfactant to be combined with the component (a1) is not limited. For example, a glycerin fatty acid ester, a diglycerin fatty acid ester, a polyglycerin fatty acid ester, a sorbitan fatty acid ester, an alkyl glyceryl ether, a POE alkyl ether, a POE sorbitan fatty acid ester, a POE glyceryl ether fatty acid ester, a POE fatty acid ester, an alkyl glucoside, and the like can be used singly or in combination of two or more.

When these nonionic surfactants are used, they are combined with the component (a1), and the HLB value (mixed HLB value) is adjusted to from 9.5 to 12.5. If the HLB value is less than 9.5, dispersibility in water deteriorates, and makeup is more likely to remain. If the HLB value exceeds 12.5, makeup tends to remain in recesses due to poor penetration into the makeup.

As the component (A), it is preferable to use, in addition to the component (a1), a single-chain type nonionic surfactant having one chain hydrocarbon group. Multi-chain type nonionic surfactants, such as the component (a1), are generally difficult to dissolve in water, and take actions such as temporary thickening due to contamination with a small amount of water. Therefore, a certain period of time is needed for complete removal when water flow is applied. When a single-chain nonionic surfactant is used in combination therewith, the aggregation of the nonionic surfactants can be suppressed, and makeup dispersibility can be improved. Improvement of makeup dispersibility refers to improved removal of makeup falling into deep holes, or removal in a short period of time during rinsing, or both of them.

The single-chain type nonionic surfactant is preferably a combination of one or more selected from the group consisting of:

(a2) a single-chain type nonionic surfactant with an HLB value of from 11 to 18 having one chain hydrocarbon group, and
(a3) a single-chain type nonionic surfactant with an HLB value of from 3 to 10 having one chain hydrocarbon group.

It is more preferable to use (a2) and (a3) in combination.

Examples of the component (a2) include a POE fatty acid ester, a POE alkyl ether, a POE sorbitan fatty acid ester, a POE glyceryl ether fatty acid ester, a polyglycerin fatty acid ester, an alkyl glucoside, and the like. The single-chain type nonionic surfactant as the component (a2) is preferably a relatively short-chain hydrocarbon group having 8 to 14 carbon atoms, from the viewpoint of suppressing the temporary thickening of the component (a1) due to water contamination during rinsing, and further promoting dispersion in water.

Examples of the component (a3) include a single-chain type glycerin fatty acid ester, a diglycerin fatty acid ester, an alkyl glyceryl ether, a sorbitan fatty acid ester, and the like.

The single-chain type nonionic surfactant as the component (a3) is preferably selected from the group consisting of a glycerin fatty acid ester, a diglycerin fatty acid ester, a glyceryl ether, and a sorbitan fatty acid ester of oleic acid or isostearic acid, from the viewpoint of stably dissolving the component (a2) in the oil phase.

As the component (A), from the viewpoint of improving dispersibility during rinsing, it is preferable to contain the component (a1) and one or more selected from the group consisting of the components (a2) and (a3), and it is more preferable to use the components (a1), (a2), and (a3) in combination.

When the components (a1), (a2), and (a3) are used in combination, the mass ratio of the component (a1) to the total amount of the components (a2) and (a3), (a1)/[(a2)+(a3)], is preferably 0.2 or more, and more preferably 0.5 or more, and is preferably 5 or less, more preferably 4.5 or less, and further more preferably 4 or less, from the viewpoint of improving makeup dispersibility. Further, the mass ratio of the component (a1) to the total amount of the components (a2) and (a3), (a1)/[(a2)+(a3)], is preferably from 0.2 to 5, more preferably from 0.2 to 4.5, and further more preferably from 0.5 to 4.

To provide a mist-type cleansing composition filled in a container equipped with a mist-type dispenser, it is necessary to control the viscosity of the cleansing composition low. For this reason, the mass ratio of the component (a1) to the total amount of the components (a2) and (a3), (a1)/[(a2)+(a3)], is preferably 0.3 or more, and more preferably 0.4 or more, and is preferably 1 or less, and more preferably 0.8 or less, from the viewpoint of reducing viscosity. Further, the mass ratio of the component (a1) to the total amount of the components (a2) and (a3), (a1)/[(a2)+(a3)], is preferably from 0.3 to 1, and more preferably from 0.4 to 0.8.

The content of the component (A) is 7 mass% or more, and preferably 10 mass% or more, in the entire composition, from the viewpoint of exhibiting good makeup removability (ability to remove makeup from the skin or substrate), and is 35 mass% or less, and more preferably 25 mass% or less, from the viewpoint of the effect of penetration into recesses (action of deep penetration into makeup entering recesses). Further, the content of the component (A) is from 7 to 35 mass%, and preferably from 10 to 25 mass%, in the entire composition.

The component (B) is an oil phase having a viscosity at 25°C of 11 mPa·s or less.

The oil phase as the component (B) acts to dissolve oil-soluble polymers which form a hard film on the foundation coating film surface, and to soften the coating film itself by penetrating into the coating film, and also serves to allow the component (A) to quickly penetrate into the interior of the structure, such as recesses. From that point of view, the viscosity at 25°C is adjusted to 11 mPa·s or less.

The viscosity is measured by using a B-type viscometer (TVB-10 Viscometer, manufactured by Toki Sangyo Co., Ltd.), and a viscosity of less than 100 mPa·s at 25°C is measured with a rotor No. 1 (number of rotations) at 60 rpm for a measurement time of 1 minute. A viscosity of from 100 to 199 mPa·s is measured with a rotor No. 1 (number of rotations) at 30 rpm, and a viscosity of from 200 to 499 mPa·s is measured with a rotor No. 1 (number of rotations) at 12 rpm.

The oil agent which constitutes the oil phase as the component (B) may be a liquid oil generally used for cosmetics, and examples thereof include hydrocarbon oils, such as isododecane, isohexadecane, light liquid isoparaffin, liquid isoparaffin, and liquid paraffin; ether oils, such as dicaprylyl ether and alkyl-1,3-dimethylbutyl ether; monoester oils, such as cetyl 2-ethylhexanoate, cetyl octanoate, isononyl isononanoate, isotridecyl isononanoate, hexyl laurate, isopropyl myristate, octyldodecyl myristate, myristyl myristate, 2-hexyldecyl myristate, isopropyl palmitate, and octyl palmitate; diester oils, such as propylene glycol dicaprylate and neopentyl glycol dicaprylate; triester oils, such as glycerin tri(caprylic/capric acid), glycerin trioleate, and glycerin tri-2-ethylhexanoate; vegetable oils, such as olive oil and jojoba oil; and the like.

The oil phase as the component (B) with lower viscosity exhibits higher performance, from the viewpoint of coating film solubility and penetration. The viscosity of the oil phase at 25°C is 11 mPa·s or less, preferably 10 mPa·s or less, and more preferably 8 mPa·s or less.

In addition, to provide a mist-type cleansing composition filled in a container equipped with a mist-type dispenser, the viscosity of the oil phase as the component (B) at 25°C is preferably 10 mPa·s or less, and more preferably 8 mPa·s or less, from the viewpoint of reducing viscosity.

Although it is possible to use an oil agent alone as the component (B), it is preferable to use an oil agent (b1) having a viscosity at 25°C of 8 mPa·s or less to adjust the viscosity. Such an oil agent has high coating film solubility and penetration. For example, even in combination with an oil agent having a viscosity of 12 mPa·s or more, it is possible to remove the foundation fallen deeply in the recesses by adjusting the viscosity in combination with these.

Examples of the oil agent (b1) include isododecane, isohexadecane, hydrogenated polyisobutene with a viscosity of 8 mPa·s or less, light liquid isoparaffin, isononyl isononanoate, isopropyl myristate, and the like.

The oil agent (b1) is preferably contained in an amount of 40 mass% or more, more preferably 50 mass% or more, and further more preferably 75 mass% or more, in the oil phase as the component (B).

Even if the viscosity of the entire oil phase is the same, a larger mixing amount of low-viscosity oil agent, such as (b1), results in more excellent removal of the foundation from deeper recesses.

From the viewpoint of excellent coating film solubility and penetration and makeup washability during rinsing, the content of the component (B) is 60 mass% or more, and preferably 65 mass% or more, and is 90 mass% or less, preferably 85 mass% or less, and more preferably 80 mass% or less, in the entire composition. Further, the content of the component (B) is from 60 to 90 mass%, preferably from 60 to 85 mass%, and more preferably from 65 to 80 mass%, in the entire composition.

The component (C) is one or more selected from the group consisting of water, an alcohol having 2 or 3 carbon atoms, and a dihydric alcohol. The component (C) increases the dissolution stability of the component (A) in the system, and simultaneously activates the function as a surfactant and promotes penetration into the foundation coating film and dispersion.

Examples of the alcohol having 2 or 3 carbon atoms include ethanol, isopropyl alcohol, and the like.

Examples of the dihydric alcohol include dipropylene glycol, butylene glycol, propanediol, and the like.

The component (C) is preferably water, ethanol, 1,3-butylene glycol, or dipropylene glycol.

The components (C) can be used alone or in combination of two or more, and the content thereof is 0.05 mass% or more, and preferably 0.5 mass% or more, in the entire composition, from the viewpoint of the removal of makeup entering recesses, and is 8 mass% or less, and preferably 7 mass% or less, from the viewpoint of rinseability and uniformity. Further, the content of the component (C) is preferably from 0.05 to 8 mass%, and more preferably from 0.5 to 7 mass%, in the entire composition.

To provide a mist-type cleansing composition filled in a container equipped with a mist-type dispenser, the content of the component (C) is preferably 0.05 mass% or more, and more preferably 0.5 mass% or more, in the entire composition, from the viewpoint of dissolution stability, and is preferably 5 mass% or less, and more preferably 4 mass% or less, from the viewpoint of maintaining low viscosity at low temperatures. To provide a mist-type cleansing composition filled in a container equipped with a mist-type dispenser, the content of the component (C) is preferably from 0.05 to 5 mass%, and more preferably from 0.5 to 4 mass%, in the entire composition.

In the present invention, a mass ratio of the component (C) to the component (A), (C)/(A), is preferably from 0.03 to 0.4, and more preferably from 0.05 to 0.3, from the viewpoint of the penetration of the component (A) into the coating film and dispersibility during rinsing.

In addition to the above components, the cleansing composition of the present invention may further contain components used for general cleansing compositions, such as surfactants other than the component (A), oily components other than the component (B), polymer compounds, UV absorbers, antioxidants, fragrances, bactericides, preservatives, antifouling agents, and the like.

The cleansing composition of the present invention can be produced by mixing and stirring each component using a general method.

The cleansing composition of the present invention is a liquid oil formulation, and is preferably in a one-liquid phase. Liquid means that the composition can be easily spilled when it is taken in the palm of the hand and tilted, and the one-liquid phase is a state with a transparent to translucent appearance and no obvious interface in the system.

The mechanism of action of general oil-based makeup removers includes the process by which the composition dissolves and loosen makeup by physical force, such as rubbing with fingers a robust surface composed of oil-soluble polymers (e.g., trimethylsiloxysilicate) which form a long-lasting makeup coating film, and the process by which the surfactant emulsifies and pulls the makeup-dispersed oil agent away from the skin by mixing with water by hand during rinsing. That is, the involvement of physical force by human hands was required not only to dissolve and disperse the makeup coating film, but also to emulsify and rinse off the cleaning agent.

By using the components (A), (B), and (C) in combination, the cleansing composition of the present invention can spontaneously permeate the foundation coating film or the like, without any physical force, simply by dropping it on the coating film, and can disperse makeup from small recesses such as pores, and rinse it off simply by exposure to water flow without rubbing.

As with general oil-based makeup removers, the cleansing composition of the present invention can be filled in a container equipped with a pump dispenser. In that case, it is difficult to apply it to the face without touching by hand; thus, it is taken on the palm of the hand and spread as needed, and water is sprayed directly using a shower, whereby makeup can be removed from the recesses such as pores.

Further, when the cleansing composition of the present invention is filled in a container equipped with a mist-type dispenser or an aerosol container, it is possible to spray it directly on the skin and remove makeup almost without touching the skin.

To provide a mist-type cleansing composition filled in a container equipped with a mist-type dispenser, it is preferable to adjust the viscosity at 25°C of the cleansing composition to 30 mPa·s or less, from the viewpoint of sprayability. Assuming winter use, it is more preferable to adjust the viscosity at 7°C to 30 mPa·s or less. In the present specification, "sprayability" means that the agent is sprayed as fine particles.

The cleansing composition of the present invention can be preferably used to remove oily stains from hydrophobic surfaces (human skin and clothing surfaces or hard surfaces), preferably used for skin cleansing, and particularly preferably used as a facial cleanser, a makeup remover, a whole-body cleanser for removing oily stains such as sunscreens, or the like.

Regarding the embodiments described above, the present invention further discloses the following cleansing composition.

<1> A cleansing composition comprising the following components (A), (B), and (C):

(A) 7 to 35 mass% of a nonionic surfactant comprising (a1) a multi-chain type nonionic surfactant which has two or more of at least one selected from the group consisting of an unsaturated chain hydrocarbon group and a saturated branched chain hydrocarbon group, and having an HLB value (a mixed HLB value when two or more nonionic surfactants are contained) of from 9.5 to 12.5;
(B) 60 to 90 mass% of an oil phase having a viscosity at 25°C of 11 mPa·s or less; and
(C) 0.05 to 8 mass% of one or more selected from the group consisting of water, an alcohol having 2 or 3 carbon atoms, and a dihydric alcohol.

<2> The cleansing composition according to <1> above, wherein a content of the component (A) is from 10 to 25 mass% in the entire composition.

<3> The cleansing composition according to <1> or <2> above, wherein the component (a1) is a multi-chain type nonionic surfactant having an HLB value of from 7 to 13, and preferably an HLB value of from 8 to 12.

<4> The cleansing composition according to any one of <1> to <3> above, wherein the unsaturated chain hydrocarbon group as the component (a1) is preferably a hydrocarbon group having 8 to 22 carbon atoms, more preferably a linear hydrocarbon group having 12 to 18 carbon atoms, and further more preferably an oleyl group.

<5> The cleansing composition according to any one of <1> to <4> above, wherein the saturated branched chain hydrocarbon group as the component (a1) is preferably a hydrocarbon group having 8 to 22 carbon atoms, more preferably a hydrocarbon group having 12 to 18 carbon atoms, and further more preferably an isostearoyl group.

<6> The cleansing composition according to any one of <1> to <5> above, wherein the component (a1) is one or more selected from the group consisting of a POE sorbitan fatty acid ester, a POE sorbitol fatty acid ester, a POE glyceryl ether fatty acid ester, and a POE fatty acid ester.

<7> The cleansing composition according to any one of <1> to <6> above, wherein the component (a1) is one or more selected from the group consisting of a POE sorbitan fatty acid ester, a POE sorbitol fatty acid ester, a POE glyceryl ether fatty acid ester, and a POE fatty acid ester, each of which has 2 to 4 oleyl groups or isostearoyl groups.

<8> The cleansing composition according to any one of <1> to <7> above, wherein the component (A) comprises a single-chain type nonionic surfactant having one chain hydrocarbon group in addition to the component (a1).

<9> The cleansing composition according to any one of <1> to <8> above, wherein the component (A) comprises, in addition to the component (a1), one or more selected from the group consisting of:

(a2) a single-chain type nonionic surfactant with an HLB value of from 11 to 18 having one chain hydrocarbon group, and

(a3) a single-chain type nonionic surfactant with an HLB value of from 3 to 10 having one chain hydrocarbon group.

<10> The cleansing composition according to <9> above, wherein the hydrocarbon group as the component (a2) has 8 to 14 carbon atoms.

<11> The cleansing composition according to <9> or <10> above, wherein the component (a3) is one or more selected from the group consisting of a glycerin fatty acid ester, a diglycerin fatty acid ester, a glyceryl ether, and a sorbitan fatty acid ester of oleic acid or isostearic acid.

<12> The cleansing composition according to any one of <9> to <11> above, wherein a mass ratio of the component (a1) to the total amount of the components (a2) and (a3), (a1)/[(a2)+(a3)], is from 0.2 to 5, preferably from 0.2 to 4.5, and more preferably from 0.5 to 4.

<13> The cleansing composition according to any one of <1> to <12> above, wherein a content of the component (B) is from 60 to 85 mass%, and preferably from 65 to 80 mass%, in the entire composition.

<14> The cleansing composition according to any one of <1> to <13> above, wherein the oil phase as the component (B) has a viscosity of 10 mPa·s or less, and preferably 8 mPa·s or less.

<15> The cleansing composition according to any one of <1> to <14> above, wherein the component (B) comprises (b1) an oil agent having a viscosity at 25°C of 8 mPa·s or less.

<16> The cleansing composition according to any one of <1> to <15> above, wherein the component (B) comprises (b1) one or more selected from the group consisting of hydrogenated polyisobutene, light liquid isoparaffin, and an isononyl isononanoate oil agent, each of which has a viscosity at 25°C of 8 mPa·s or less.

<17> The cleansing composition according to any one of <1> to <16> above, wherein the component (B) comprises (b1) an oil agent having a viscosity at 25°C of 8 mPa·s or less in an amount of 40 mass% or more, preferably 50 mass% or more, and more preferably 75 mass% or more.

<18> The cleansing composition according to any one of <1> to <17> above, wherein a content of the component (C) is from 0.5 to 7 mass% in the entire composition.

<19> The cleansing composition according to any one of <1> to <18> above, wherein the component (C) is one or more selected from the group consisting of water, ethanol, 1,3-butylene glycol, and dipropylene glycol.

<20> The cleansing composition according to any one of <1> to <19> above, wherein a mass ratio of the component (C) to the component (A), (C)/(A), is from 0.03 to 0.4, and preferably from 0.05 to 0.3.

<21> The cleansing composition according to any one of <1> to <20> above, preferably for use in skin cleaning, and more preferably facial cleaning, makeup removal, or whole-body cleaning.

<22> The cleansing composition according to any one of <1> to <21> above, which is filled in a container equipped with a mist-type dispenser or an aerosol container, for use in spraying on the skin.

Examples

Test Example 1

A cleansing composition with the formulation shown in Table 1 (Example A) and a commercially available makeup remover (Biore Perfect Oil, manufactured by Kao Corporation) were evaluated for the removal of makeup fallen into deep holes, and the removal of makeup on a model sheet. The results are also shown in Table 1.

(Production method)

The component (A) is weighed. When two or more components are used, they are mixed well. Then, the component (B) was added while stirring, and finally the component (C) was added dropwise, followed by stirring until homogeneous, thereby obtaining a cleansing composition (Example A).

(Evaluation method)

(1) Removal of makeup fallen into deep holes:

The method is to fill intentionally created fine recesses with a foundation to form a thick foundation coating film, and to apply the agent thereto, followed by rinsing off. Since this method can strongly reflect the high penetration of the cleansing composition into the recesses, it is possible to measure the removability in the depth direction for the foundation having entered the recesses.

To the vinyl chloride sheet-like substrate (manufactured by Komatsu Ltd.) with 61 holes of 0.5 mm diameter and 0.5 mm depth evenly distributed in a circular area of 32 mm diameter shown in Fig. 1, a long-lasting foundation (REVLON Color Stay Makeup Foundation) is applied to fill the holes, and the makeup protruding from the surface is smoothed out and scraped off, followed by drying for over 3 hours.

0.5 mL of each cleansing composition is dropped onto the above substrate, left for 1 minute, and then rinsed off for 20 seconds by directly applying water from a shower. After drying, magnified observation was performed by DSA to evaluate the removal of the foundation from the halls based on the following criteria.

A rating of "3" or higher is considered acceptable.

1: No change was observed in the shape of the foundation in the holes.
2: The foundation in the holes was slightly removed in comparison with before application.
3: The foundation in the holes was clearly removed in comparison with before application.
4: The foundation in the holes was removed to the extent that the shape of the holes in the substrate could be confirmed.
5: The foundation was removed from the holes, exposing the substrate.

(2) Removal of makeup on model sheet:

The method is to form a general amount of foundation coating film on a model sheet with fine irregularities similar to those of the actual skin, and to measure the removal of the foundation coating film comprehensively. This method can evaluate the removability and rinseability of the foundation in actual use.

0.045 g of a long-lasting foundation (REVLON Color Stay Makeup Foundation) is applied to an area of $10 \times 5\,cm^2$ on a model sheet having fine pore-like recesses (manufactured by Okamoto Kaseihin Co., Ltd., artificial leather, Laforet White), and dried for over 1 hour.

The foundation applied site is cut into small pieces of $2 \times 2.5\,cm^2$ to form test pieces.

One drop of each cleansing composition is dropped on the test pieces with a dropper and left for 1 minute. After rinsing with a shower for a predetermined period of time (as shown in the table) and drying, the removal of the foundation was visually evaluated based on the following criteria.

A rating of "A", "B", or "C" is considered acceptable.

E: No change was observed in the coating film of the foundation.
D: Although the coating film of the foundation was slightly removed, most of the coating film remained.
C: The coating film of the foundation was clearly removed.
B: The coating film of the foundation was almost removed.
A: The coating film of the foundation was completely removed.

[Table 1]

| | | Component name | Raw material name | Example A | Commercial product A | Commercial product A |
|---|---|---|---|---|---|---|
| | B | Oil agent mixture (Light liquid isoparaffin/isononyl isononanoate = 1/1) | Viscosity 6 mPa·s: PARLEAM 4 (manufactured by NOF Corporation)/SALACOS 99 (manufactured by The Nisshin OilliO Group, Ltd.) | 75 | | |
| | A-a1 | Polyoxyethylene(30) sorbitol tetraoleate | HLB 10.5: RHEODOL 430 (manufactured by Kao Corporation) | 12 | | |
| | A-a2 | Polyoxyethylene(12) lauric acid ester | HLB 14: EMANON 1112 (manufactured by Kao Corporation) | 9 | | |
| | A-a3 | Sorbitan isostearate | HLB 4.7: NIKKOL SI-10RV (manufactured by Nippon Surfactant Industries Co., Ltd.) | 3 | | |
| | C | Water | | 1 | | |
| | | Total | | 100 | | |
| | | Mixed HLB of component (A) | | 11.0 | | |

(continued)

|  | Component name | Raw material name | Example A | Commercial product A | Commercial product A |
|---|---|---|---|---|---|
| Evaluation conditions | | | Left for 1 min → rinsed off | Left for 1 min → rinsed off | Massaged with fingers 10 times → rinsed off |
| Removal of makeup fallen into deep holes | | | 4 | 1 | 2 |
| Removal of makeup on model sheet (rinsed for 10 sec) | | | A | E | B-C |

Examples 1 to 34 and Comparative Examples 1 to 15

Cleansing compositions with the formulations shown in Tables 2 to 9 were produced in the same manner as in Example A, and the removal of makeup falling into deep holes, and the removal of makeup on a model sheet were evaluated. The results are also shown in Tables 2 to 9.

[Table 2]

| | Component name | Raw material name | Example 1 | Example 2 | Example 3 | Example 4 | Example 5 | Example 6 | Example 7 | Comparative Example 1 |
|---|---|---|---|---|---|---|---|---|---|---|
| B | Oil agent mixture (Light liquid isoparaffin/isononyl isononanoate = 1/1) | Viscosity 6 mPa·s: PARLEAM 4 (manufactured by NOF Corporation)/SALACOS 99 (manufactured by The Nisshin OilliO Group, Ltd.) | 79 | 79 | 79 | 79 | 79 | 79 | 79 | 79 |
| A | Polyoxyethylene(20) sorbitan trioleate | HLB 11: RHEODOL TW-O320 (manufactured by Kao Corporation) | 20 | | | | | | | |
| | Polyoxyethylene(30) sorbitol tetraoleate | HLB 10.5: RHEODOL 430 (manufactured by Kao Corporation) | | 20 | | | | | | |
| | Polyoxyethylene(40) sorbitol tetraoleate | HLB 11.8: RHEODOL 440 (manufactured by Kao Corporation) | | | 20 | | | | | |
| | Polyoxyethylene(30) glyceryl triisostearate | HLB 10: EMALEX GWIS330 (manufactured by Nihon Emulsion Co., Ltd.) | | | | 20 | | | | |
| | Polyoxyethylene(20) glyceryl triisostearate | HLB 11: M FINE OIL ISG-20T (manufactured by Miyoshi Oil & Fat Co., Ltd.) | | | | | 20 | | | |
| | Polyoxyethylene(20) glyceryl diisostearate | HLB 10: EMALEX GWIS220 (manufactured by Nihon Emulsion Co., Ltd.) | | | | | | 20 | | |
| | Polyoxyethylene(12) diisostearic acid ester | HLB 10: NONION D-IS600 | | | | | | | 20 | |
| | Polyoxyethylene(10) glyceryl isostearate | HLB 10: EMALEX GWIS110 (manufactured by Nihon Emulsion Co., Ltd.) | | | | | | | | 20 |
| C | Water | | 1 | 1 | 1 | 1 | 1 | 1 | 1 | 1 |
| | Total | | 100 | 100 | 100 | 100 | 100 | 100 | 100 | 100 |
| | Number of oleyl groups or isostearoyl groups in component (A) | | 3 | 4 | 4 | 3 | 3 | 2 | 2 | 1 |
| | HLB of component (A) | | 11 | 10.5 | 11.8 | 10 | 11 | 10 | 10 | 10 |
| | Removal of makeup fallen into deep holes | | 3 | 3.5 | 3 | 3.5 | 3 | 3 | 3 | 1.5 |
| | Removal of makeup on model sheet (rinsed for 10 sec) | | C | B | A | A | A | A | A | C |

[Table 3]

| | Component name | Raw material name | Comparative Example 2 | Example 8 | Example 9 | Example 10 | Comparative Example 3 |
|---|---|---|---|---|---|---|---|
| B | Oil agent mixture (Light liquid isoparaffin/isononyl isononanoate = 1/1) | Viscosity 6 mPa·s: PARLEAM 4 (manufactured by NOF Corporation)/SALACOS 99 (manufactured by The Nisshin OilliO Group, Ltd.) | 79 | 78.8 | 78.8 | 78.5 | 78.5 |
| A | Polyoxyethylene(12) lauric acid ester | HLB 14: EMANON 1112 (manufactured by Kao Corporation) | 2 | 5 | 8 | 9 | 10 |
| | Polyoxyethylene(30) sorbitol tetraoleate | HLB 10.5: RHEODOL 430 (manufactured by Kao Corporation) | 10 | 10 | 10 | | |
| | Polyoxyethylene(40) sorbitol tetraoleate | HLB 11.8: RHEODOL 440 (manufactured by Kao Corporation) | | | | 10 | 10 |
| | Polyglyceryl-2 isostearate | HLB 6: COSMOL 41V (manufactured by The Nisshin OilliO Group, Ltd.) | 8 | 5 | 2 | 1 | |
| C | Water | | 1 | 1.2 | 1.2 | 1.5 | 1.5 |
| | Total | | 100 | 100 | 100 | 100 | 100 |
| | Mixed HLB of component (A) | | 9.05 | 10.25 | 11.45 | 12.5 | 13 |
| | Removal of makeup fallen into deep holes | | 3 | 4 | 4 | 3 | 2 |
| | Removal of makeup on model sheet (rinsed for 10 sec) | | D | C | A | A | A |

EP 4 613 257 A1

[Table 4]

| | Component name | Raw material name | Comparative Example 4 | Comparative Example 5 | Example 11 | Example 12 | Example 13 | Comparative Example 6 | Comparative Example 7 | Comparative Example 8 |
|---|---|---|---|---|---|---|---|---|---|---|
| B | Oil agent mixture (Light liquid isoparaffin/isononyl isononanoate = 1/1) | Viscosity 6 mPa·s: PARLEAM 4 (manufactured by NOF Corporation)/-SALACOS 99 (manufactured by The Nisshin OilliO Group, Ltd.) | 96 | 94 | 89 | 79 | 69 | 59 | 49 | 39 |
| A | Polyoxyethylene(30) sorbitol tetraoleate | HLB 10.5: RHEODOL 430 (manufactured by Kao Corporation) | 3 | 5 | 10 | 20 | 30 | 40 | 50 | 60 |
| C | Water | | 1 | 1 | 1 | 1 | 1 | 1 | 1 | 1 |
| | Total | | 100 | 100 | 100 | 100 | 100 | 100 | 100 | 100 |
| | HLB of component (A) | | 10.5 | 10.5 | 10.5 | 10.5 | 10.5 | 10.5 | 10.5 | 10.5 |
| | Content of component (A) | | 3 | 5 | 10 | 20 | 30 | 40 | 50 | 60 |
| | Removal of makeup fallen into deep holes | | 3 | 3.5 | 4 | 4 | 3 | 2 | 2 | 2 |
| | Removal of makeup on model sheet (rinsed for 10 sec) | | D | D | C | B | B | C | D | E |

[Table 5]

| | Component name | Raw material name | Example 14 | Example 15 | Example 16 | Comparative Example 9 | Comparative Example 10 | Comparative Example 11 |
|---|---|---|---|---|---|---|---|---|
| B | Light liquid isoparaffin | Viscosity 5 mPa·s: PARLEAM 4 (manufactured by NOF Corporation) | 39 | 29 | 19 | 10 | 5 | |
| B | Isononyl isononanoate | Viscosity 7 mPa·s: SALACOS 99 (manufactured by The Nisshin OilliO Group, Ltd.) | 40 | 30 | 20 | 10 | 5 | |
| B | Cetyl 2-ethylhexanoate | Viscosity 14 mPa·s: EXCEPARL HO (manufactured by Kao Corporation) | | 10 | 20 | 30 | 35 | 40 |
| B | Liquid paraffin | Viscosity 18 mPa·s: HICALL K-230 (manufactured by Kaneda Co., Ltd.) | | 10 | 20 | 29 | 34 | 39 |
| A | Polyoxyethylene(30) sorbitol tetraoleate | HLB 10.5: RHEODOL 430 (manufactured by Kao Corporation) | 20 | 20 | 20 | 20 | 20 | 20 |
| C | Water | | 1 | 1 | 1 | 1 | 1 | 1 |
| | Total | | 100 | 100 | 100 | 100 | 100 | 100 |
| | Viscosity of component (B): mPa/s | | 5.0 | 7.0 | 8.6 | 11.7 | 13.5 | 15.0 |
| | Removal of makeup fallen into deep holes | | 3.5 | 3 | 3 | 2 | 2 | 2 |
| | Removal of makeup on model sheet (rinsed for 10 sec) | | B | B | C | D | D | E |

EP 4 613 257 A1

[Table 6]

|  | | Component name | Raw material name | Example 17 | Example 18 |
|---|---|---|---|---|---|
| B | | Oil agent mixture (Light liquid isoparaffin/isononyl isononanoate = 1/1) | Viscosity 6 mPa·s: PARLEAM 4 (manufactured by NOF Corporation)/SALACOS 99 (manufactured by The Nisshin OilliO Group, Ltd.) | 34 | 69 |
| B | | Liquid paraffin | Viscosity 18 mPa·s: HICALL K-230 (manufactured by Kaneda Co., Ltd.) | 45 | |
| B | | Liquid paraffin | Viscosity 135 mPa·s: HICALL K-350 (manufactured by Kaneda Co., Ltd.) | | 10 |
| A | | Polyoxyethylene(12) lauric acid ester | HLB 14: EMANON 1112 (manufactured by Kao Corporation) | 7 | 7 |
| A | | Polyoxyethylene(30) sorbitol tetraoleate | HLB 10.5: RHEODOL 430 (manufactured by Kao Corporation) | 10 | 10 |
| A | | Polyglyceryl-2 isostearate | HLB 6: COSMOL 41V (manufactured by The Nisshin OilliO Group, Ltd.) | 3 | 3 |
| C | | | Water | 1 | 1 |
| | Total | | | 100 | 100 |
| | Viscosity of component (B): mPa/s | | | 9.7 | 10.3 |
| | Removal of makeup fallen into deep holes | | | 3 | 4 |
| | Removal of makeup on model sheet (rinsed for 10 sec) | | | A-B | A-B |

[Table 7]

| | Component name | Raw material name | Comparative Example 12 | Example 19 | Example 20 | Comparative Example 13 | Example 21 | Example 22 | Example 23 | Comparative Example 14 |
|---|---|---|---|---|---|---|---|---|---|---|
| B | Oil agent mixture (Light liquid isoparaffin/isononyl isononanoate = 1/1) | Viscosity 5 mPa·s: PARLEAM 4 (manufactured by NOF Corporation)/-SALACOS 99 (manufactured by The Nisshin OilliO Group, Ltd.) | 80 | 79 | 75 | 70 | 79 | 73 | 79 | 79 |
| A | Polyoxyethylene(30) sorbitol tetraoleate | HLB 10.5: RHEODOL 430 (manufactured by Kao Corporation) | 20 | 20 | 20 | 20 | 20 | 20 | 20 | 20 |
| C | 1,3-Butylene glycol | 1.3-BG-P (manufactured by KH Neochem Co., Ltd.) | | | | | 1 | 7 | | |
| | Ethanol | Ethanol (Japanese Pharmacopoeia, fermentation) (manufactured by Japan Alcohol Corporation) | | | | | | | 1 | |
| | Water | | | 1 | 5 | 10 | | | | |
| | Glycerin | Glycerin 99.5% (USP) (manufactured by IOI Acidchem Sdn. Bhd.) | | | | | | | | 1 |
| | Total | | 100 | 100 | 100 | 100 | 100 | 100 | 100 | 100 |
| | Content of component (C) | | 0 | 1 | 5 | 10 | 1 | 7 | 1 | 0 |
| | C/A | | - | 0.05 | 0.25 | 0.5 | 0.05 | 0.35 | 0.05 | - |
| | Removal of makeup fallen into deep holes | | 2 | 3.5 | 3.5 | 3 | 3.5 | 3 | 3 | 3 |
| | Removal of makeup on model sheet (rinsed for 10 sec) | | C | B | B | D | B | B | B | D |

[Table 8]

| | Component name | Raw material name | Example 24 | Example 25 | Example 26 | Example 27 | Example 28 | Example 29 | Example 30 |
|---|---|---|---|---|---|---|---|---|---|
| B | Oil agent mixture (Light liquid isoparaffin/isononyl isononanoate = 1/1) | Viscosity 6 mPa·s: PARLEAM 4 (manufactured by NOF Corporation)/SALACOS 99 (manufactured by The Nisshin OilliO Group, Ltd.) | 79 | 79 | 79 | 79 | 79 | 79 | 78.8 |
| A-a1 | Polyoxyethylene(30) sorbitol tetraoleate | HLB 10.5: RHEODOL 430 (manufactured by Kao Corporation) | 10 | 10 | 10 | 10 | 10 | 10 | |
| | Polyoxyethylene(30) glyceryl triisostearate | HLB 10: EMALEX GWIS330 (manufactured by Nihon Emulsion Co., Ltd.) | | | | | | | 10 |
| A-a2 | Polyoxyethylene(12) lauric acid ester | HLB 14: EMANON 1112 (manufactured by Kao Corporation) | 7 | | | | 8 | 8 | |
| | Polyoxyethylene(6) (caprylic/capric acid) glyceryl | HLB 15: TEGOSOFT GMC 6 MB (manufactured by Evonik Operations GmbH) | | 7 | | | | | |
| | Polyoxyethylene(7) coconut oil fatty acid glyceryl | HLB 13: LEVENOL C-301 (manufactured by Kao Corporation) | | | 8.5 | | | | 9 |
| | Polyoxyethylene(9) lauryl ether | HLB 13.6: EMULGEN 109P (manufactured by Kao Corporation) | | | | 8.5 | | | |
| | Polyoxyethylene(60) sorbitol tetraoleate | HLB 13.8: RHEODOL 460 (manufactured by Kao Corporation) | | | | | | | |
| A-a3 | Polyglyceryl-2 isostearate | HLB 6: COSMOL 41V (manufactured by The Nisshin OilliO Group, Ltd.) | 3 | 3 | 1.5 | 1.5 | | | |
| | Sorbitan isostearate | HLB 4.7: NIKKOL SI-10RV (manufactured by Nippon Surfactant Industries Co., Ltd.) | | | | | 2 | | |
| | Glyceryl isostearate | HLB 5: GWIS100 (manufactured by Nihon Emulsion Co., Ltd.) | | | | | | 2 | 1 |
| C | Water | | 1 | 1 | 1 | 1 | 1 | 1 | 1.2 |
| | Total | | 100 | 100 | 100 | 100 | 100 | 100 | 100 |
| | Mixed HLB of component (A) | | 11.1 | 11.2 | 11.0 | 11.2 | 11.0 | 11.0 | 11.1 |
| | Removal of makeup fallen into deep holes | | 4 | 4 | 4 | 4 | 4 | 4 | 4 |

16

| Component name | Raw material name | Example 24 | Example 25 | Example 26 | Example 27 | Example 28 | Example 29 | Example 30 |
|---|---|---|---|---|---|---|---|---|
| Removal of makeup on model sheet (rinsed for 10 sec) | | B | B | B | A | A | A | A |
| Removal of makeup on model sheet (quickly passing under shower 5 times) | | B-C | C | B | A-B | B-C | B-C | B |

[Table 9]

|  | Component name | Raw material name | Example 31 | Example 32 | Example 33 | Example 34 | Comparative Example 15 |
|---|---|---|---|---|---|---|---|
| B | Light liquid isoparaffin/isononyl isononanoate = 1/1 mixed oil agent | Viscosity 6 mPa·s: PARLEAM 4 (manufactured by NOF Corporation)/SALACOS 99 (manufactured by The Nisshin OilliO Group, Ltd.) | 79 | 79 | 79 | 79 | 79 |
| A-a1 | Polyoxyethylene(30) sorbitol tetraoleate | HLB 10.5: RHEODOL 430 (manufactured by Kao Corporation) | 15 | 10 | 6.5 | 5 | |
| A-a2 | Polyoxyethylene(12) lauric acid ester | HLB 14: EMANON 1112 (manufactured by Kao Corporation) | 4.5 | 7 | 9.5 | 10 | 13 |
| A-a3 | Polyglyceryl-2 isostearate | HLB 6: COSMOL 41V (manufactured by The Nisshin OilliO Group, Ltd.) | 0.5 | 3 | 4 | 5 | 7 |
| C | Water | | 1 | 1 | 1 | 1 | 1 |
| | Total | | 100 | 100 | 100 | 100 | 100 |
| | Mixed HLB of component (A) | | 11.2 | 11.1 | 11.3 | 11.1 | 11.2 |
| | (a1)/[(a2)+(a3)] | | 3.0 | 1.0 | 0.5 | 0.3 | 0.0 |
| | Removal of makeup falling into deep holes | | 4 | 4 | 4 | 4 | 2 |
| | Removal of makeup on model sheet (rinsed for 10 sec) | | A | A-B | A-B | A-B | C-D |
| | Removal of makeup on model sheet (quickly passing under shower 5 times) | | B | A-B | B | C | D |

Formulation Example 1

A cleansing composition with the formulation shown in Table 10 was produced in the same manner as in Example A.

This cleansing composition could completely remove the foundation just by spreading it all over the face with hands and rinsing off. The fact that the foundation was completely removed was confirmed by wiping off with a wipe-off lotion after rinsing.

[Table 10]

|  | Component name | Raw material name | mass% |
|---|---|---|---|
| B | Isohexadecane | Viscosity 4 mPa·s: PERMETYL 101A (manufactured by INEOS Manufacturing Deutschland GmbH) | 14 |
| B | Liquid paraffin | Viscosity 7 mPa·s: HICALL K-140N (manufactured by Kaneda Co., Ltd.) | 6 |
| B | Isopropyl myristate | Viscosity 7 mPa·s: EXCEPARL IPM (manufactured by Kao Corporation) | 15 |
| B | Isononyl isononanoate | Viscosity 7 mPa·s: SALACOS 99 (manufactured by The Nisshin OilliO Group, Ltd.) | 25 |
| B | Isotridecyl isononanoate | Viscosity 11 mPa·s: SALACOS 913 (manufactured by The Nisshin OilliO Group, Ltd.) | 6 |
| B | Dimethylpolysiloxane | Viscosity 10 mPa·s: SILICONE KF-96 10cs (manufactured by Shin-Etsu Chemical Co., Ltd.) | 4 |
| A-a2 | Polyoxyethylene(6) (caprylic/capric acid) glyceryl | HLB 15: TEGOSOFT GMC 6 MB (manufactured by Evonik Operations GmbH) | 5.5 |
| A-a2 | Polyoxyethylene(12) lauric acid ester | HLB 14: EMANON 1112 (manufactured by Kao Corporation) | 7.5 |
| A-a1 | Polyoxyethylene(30) glyceryl triisostearate | HLB 10: EMALEX GWIS330 (manufactured by Nihon Emulsion Co., Ltd.) | 6 |
| A-a3 | Glyceryl isostearate | HLB 5: GWIS100 (manufactured by Nihon Emulsion Co., Ltd.) | 6 |
| C | Ethanol | | 2 |
| C | Water | | 3 |
|  | Total | | 100 |

Formulation Example 2

A cleansing composition with the formulation shown in Table 11 was produced in the same manner as in Example A.

35 g of the cleansing composition was filled in a 100-mL aerosol container, and the container was filled with 15 g of LPG to form an aerosol type.

This cleansing composition was sprayed over the entire face with eyes closed, and then rinsed off by direct application of a shower. As a result, the foundation could be completely removed without touching it with hands. The fact that foundation was completely removed was confirmed by wiping off with a wipe-off lotion after rinsing.

[Table 11]

|  | Component name | Raw material name | mass% |
|---|---|---|---|
| B | Isododecane | Viscosity 3 mPa·s: MARCAZOLE R (manufactured by Maruzen Petrochemical Co., Ltd.) | 6 |
| B | Light liquid isoparaffin | Viscosity 5 mPa·s: PARLEAM 4 (manufactured by NOF Corporation) | 19.5 |

(continued)

|  | Component name | Raw material name | mass% |
|---|---|---|---|
| B | Isononyl isononanoate | Viscosity 7 mPa·s: SALACOS 99 (manufactured by The Nisshin OilliO Group, Ltd.) | 25 |
| B | Isopropyl myristate | Viscosity 7 mPa·s: EXCEPARL IPM (manufactured by Kao Corporation) | 8.5 |
| B | Cetyl 2-ethylhexanoate | Viscosity 14 mPa·s: EXCEPARL HO (manufactured by Kao Corporation) | 10 |
| A-a2 | Alkyl (8-10) glucoside | HLB 18: MYDOL 10 (manufactured by Kao Corporation) | 1.52 |
| A-a2 | Polyoxyethylene(12) lauric acid ester | HLB 14: EMANON 1112 (manufactured by Kao Corporation) | 7 |
| A-a2 | Polyoxyethylene(7) coconut oil fatty acid glyceryl | HLB 13: LEVENOL C-301 (manufactured by Kao Corporation) | 5 |
| A-a3 | Polyglyceryl-2 isostearate | HLB 6: COSMOL 41V (manufactured by The Nisshin OilliO Group, Ltd.) | 6.4 |
| A-a1 | Polyoxyethylene(20) sorbitan trioleate | HLB 11: RHEODOL TW-O320 (manufactured by Kao Corporation) | 2 |
| A-a1 | Polyoxyethylene(30) sorbitol tetraoleate | HLB 10.5: RHEODOL 430 (manufactured by Kao Corporation) | 2 |
| C | 1,3-Butylene glycol | 1.3-BG-P (manufactured by KH Neochem Co., Ltd.) | 1.28 |
| C | Water (taken over from MYDOL 10) | | 5.8 |
|  | Total | | 100 |

Formulation Example 3

A cleansing composition with the formulation shown in Table 12 was produced in the same manner as in Example A.

70 g of the cleansing composition was filled in a 100-mL container equipped with a mist-type dispenser to form a mist-type sample.

This cleansing composition was sprayed over the entire face with eyes closed, and then rinsed off by direct application of a shower. As a result, the foundation could be completely removed without touching it with hands. The foundation being completely removed was confirmed by wiping off with a wipe-off lotion after rinsing.

[Table 12]

|  | Component name | Raw material name | mass% |
|---|---|---|---|
| B | Isododecane | Viscosity 3 mPa·s: MARCAZOLE R (manufactured by Maruzen Petrochemical Co., Ltd.) | 6 |
| B | Light liquid isoparaffin | Viscosity 5 mPa·s: PARLEAM 4 (manufactured by NOF Corporation) | 16.9 |
| B | Isononyl isononanoate | Viscosity 7 mPa·s: SALACOS 99 (manufactured by The Nisshin OilliO Group, Ltd.) | 25 |
| B | Isopropyl myristate | Viscosity 7 mPa·s: EXCEPARL IPM (manufactured by Kao Corporation) | 15.8 |
| B | Cetyl 2-ethylhexanoate | Viscosity 7 mPa·s: CETIOL OE-DEO C(BASF) | 10 |
| A-a2 | Alkyl (8-10) glucoside | HLB 18: MYDOL 10 (manufactured by Kao Corporation) | 1.97 |
| A-a2 | Polyoxyethylene(12) lauric acid ester | HLB 14: EMANON 1112 (manufactured by Kao Corporation) | 6 |

(continued)

|  | Component name | Raw material name | mass% |
|---|---|---|---|
| A-a2 | Polyoxyethylene(7) coconut oil fatty acid glyceryl | HLB 13: LEVENOL C-301 (manufactured by Kao Corporation) | 2.7 |
| A-a3 | Polyglyceryl-2 isostearate | HLB 6: COSMOL 41V (manufactured by The Nisshin OilliO Group, Ltd.) | 4.7 |
| A-a1 | Polyoxyethylene(20) sorbitan trioleate | HLB 11: RHEODOL TW-O320 (manufactured by Kao Corporation) | 1.25 |
| A-a1 | Polyoxyethylene(30) sorbitol tetraoleate | HLB 10.5: RHEODOL 430 (manufactured by Kao Corporation) | 6.3 |
| C | 1,3-Butylene glycol | 1.3-BG-P (manufactured by KH Neochem Co., Ltd.) | 0.5 |
| C | Water (taken over from MYDOL 10) | | 2.88 |
| | Total | | 100 |
| | Viscosity at 25°C (mPa·s) | | 11.4 |
| | Viscosity at 7°C (mPa·s) | | 28.6 |

**Claims**

1. A cleansing composition comprising the following components (A), (B), and (C):

   (A) 7 to 35 mass% of a nonionic surfactant comprising (a1) a multi-chain type nonionic surfactant which has two or more of at least one selected from the group consisting of an unsaturated chain hydrocarbon group and a saturated branched chain hydrocarbon group, and having an HLB value (a mixed HLB value when two or more nonionic surfactants are contained) of from 9.5 to 12.5;
   (B) 60 to 90 mass% of an oil phase having a viscosity at 25°C of 11 mPa·s or less; and
   (C) 0.05 to 8 mass% of one or more selected from the group consisting of water, an alcohol having 2 or 3 carbon atoms, and a dihydric alcohol.

2. The cleansing composition according to claim 1, wherein the component (a1) is a multi-chain type nonionic surfactant having an HLB value of from 7 to 13.

3. The cleansing composition according to claim 1 or 2, wherein the component (a1) is one or more selected from the group consisting of a POE sorbitan fatty acid ester, a POE sorbitol fatty acid ester, a POE glyceryl ether fatty acid ester, and a POE fatty acid ester.

4. The cleansing composition according to any one of claims 1 to 3, wherein the component (A) comprises a single-chain type nonionic surfactant having one chain hydrocarbon group in addition to the component (a1).

5. The cleansing composition according to any one of claims 1 to 4, wherein the component (A) comprises, in addition to the component (a1), one or more selected from the group consisting of:

   (a2) a single-chain type nonionic surfactant with an HLB value of from 11 to 18 having one chain hydrocarbon group, and
   (a3) a single-chain type nonionic surfactant with an HLB value of from 3 to 10 having one chain hydrocarbon group.

6. The cleansing composition according to claim 5, wherein a mass ratio of the component (a1) to the total amount of the components (a2) and (a3), (a1)/[(a2)+(a3)], is 0.2 or more and 5 or less.

7. The cleansing composition according to any one of claims 1 to 6, wherein the component (B) comprises (b1) 40 mass% or more of an oil agent having a viscosity at 25°C of 8 mPa·s or less.

8. The cleansing composition according to any one of claims 1 to 7, wherein the component (C) is one or more selected from the group consisting of water, ethanol, 1,3-butylene glycol, and dipropylene glycol.

9. The cleansing composition according to any one of claims 1 to 8, wherein a mass ratio of the component (C) to the component (A), (C)/(A), is from 0.03 to 0.4.

10. The cleansing composition according to any one of claims 1 to 9, which is filled in a container equipped with a mist-type dispenser or an aerosol container, for use in spraying on the skin.

Fig. 1

# INTERNATIONAL SEARCH REPORT

| International application No. |
|---|
| **PCT/JP2023/038902** |

**A. CLASSIFICATION OF SUBJECT MATTER**

*A61K 8/34*(2006.01)i; *A61Q 1/14*(2006.01)i; *C11D 1/66*(2006.01)i; *C11D 1/68*(2006.01)i; *C11D 3/20*(2006.01)i
FI:  A61K8/34; A61Q1/14; C11D1/66; C11D1/68; C11D3/20

According to International Patent Classification (IPC) or to both national classification and IPC

**B. FIELDS SEARCHED**

Minimum documentation searched (classification system followed by classification symbols)

A61K8/34; A61Q1/14; C11D1/66; C11D1/68; C11D3/20

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | WO 2021/084837 A1 (TAIYO KAGAKU CO., LTD.) 06 May 2021 (2021-05-06) claims, paragraphs [0041], formulation 1 | 1-5, 8-9 |
| A | | 6-7, 10 |
| X | JP 2007-023025 A (TAIYO KAGAKU CO., LTD.) 01 February 2007 (2007-02-01) claims, paragraph [0025], table 1, example 2 | 1–2, 8–9 |
| A | | 3–7, 10 |
| X | JP 2004-256474 A (KABUSHIKI KAISHA YAKULT HONSHA) 16 September 2004 (2004-09-16) claims, paragraph [0034], table 3, products 1-2 | 1-3, 8-9 |
| X | JP 2008-308459 A (TOHO CHEMICAL INDUSTRIES CO., LTD.) 25 December 2008 (2008-12-25) claims, paragraphs [0026], [0033], table 1, compound 3, invention product 3 | 1-3, 8-9 |
| P, X | WO 2022/270350 A1 (SHISEIDO COMPANY, LTD.) 29 December 2022 (2022-12-29) claims, paragraphs [0053]-[0055], tables 1-3 | 1-3, 8-9 |

☑ Further documents are listed in the continuation of Box C.    ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **06 December 2023** | **16 January 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

## INTERNATIONAL SEARCH REPORT

| International application No. |
| --- |
| **PCT/JP2023/038902** |

**C.  DOCUMENTS CONSIDERED TO BE RELEVANT**

| Category* | Citation of document, with indication, where appropriate, of the relevant passages | Relevant to claim No. |
| --- | --- | --- |
| P, X | JP 2023-097920 A (KOSE CORP.) 10 July 2023 (2023-07-10)<br>claims, paragraph [0040], example 12 | 1-3, 8-9 |

Form PCT/ISA/210 (second sheet) (January 2015)

**INTERNATIONAL SEARCH REPORT**
Information on patent family members

International application No.

**PCT/JP2023/038902**

| Patent document cited in search report | | | Publication date (day/month/year) | Patent family member(s) | Publication date (day/month/year) |
|---|---|---|---|---|---|
| WO | 2021/084837 | A1 | 06 May 2021 | (Family: none) | |
| JP | 2007-023025 | A | 01 February 2007 | US 2009/0281199 A1 claims, table 1, example 2 | |
| JP | 2004-256474 | A | 16 September 2004 | (Family: none) | |
| JP | 2008-308459 | A | 25 December 2008 | (Family: none) | |
| WO | 2022/270350 | A1 | 29 December 2022 | (Family: none) | |
| JP | 2023-097920 | A | 10 July 2023 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002241224 A
- JP 2004238376 A